# EUROPEAN PATENT APPLICATION

(11) **EP 3 657 170 A1**
(43) Date of publication of application: **27.05.2020**
(21) Application number: 18207277.7
(22) Date of filing: 20.11.2018
(51) Int. Cl.: G01N 33/574

(54) **METHOD FOR THE EARLY DETECTION AND DIAGNOSIS OF PRIMARY AND RELAPSED OVARIAN CARCINOMA (OC)**

(71) Applicant: Philipps-Universität Marburg, 35037 Marburg (DE)
(72) Inventor: MÜLLER, Rolf, 35041 Marburg (DE); WAGNER, Uwe, 35274 Schönbach (DE)
(74) Representative: Stumpf, Peter

(57) **Abstract**

The invention describes a method and a device for identifying individuals with suspected ovarian carcinoma (OC), especially early stage and relapsed OC, in biological samples from these individuals. The method comprises the steps (i) measuring the concentration of suitable markers in a biological sample from an individual, (ii) calculating a multi-marker score, and (iii) comparing the multi-marker score of said individual with a threshold.

## Description

### Technical Field of the Invention

This invention concerns a method for the early detection of an ovarian carcinoma and for the monitoring of an ovarian carcinoma recurrence in an individual patient. OC is the common abbreviation of ovarian carcinoma. Early detection means to diagnose OC at an early stage in which no, or only minor clinical symptoms are present, or in which a clear diagnosis is not possible. This invention is therefore also a screening method for the early detection of OC. This invention also enables disease monitoring in patients with already diagnosed and treated OC to allow for the detection of relapsed disease at an early stage providing better therapy options. OC is a malignant tumour of the ovaries or fallopian tubes (oviducts) in humans or animals, and occurs as solid tumour masses and peritoneal carcinomatosis. OC and OC precursor lesions (e.g. serous tubal intraepithelial carcinoma and cortical inclusion cysts) comprises 2C73.0, 2C74, 2D90, 2D91, and XH8D74 according to ICD-11 classification. OC is usually diagnosed at an advanced stage, since suitable methods for early detection are not available. OC is the deadliest of all gynaecological malignancies and shows an overall 5-year survival rate of less than 50%. The most common form of OC is high-grade serous ovarian cancer (HGSOC). HGSOC makes up the majority of OC cases and has the lowest survival rate.

The peritoneal fluid or ascites fluid is the free fluid in the abdominal and pelvic cavities. In a large number of diseases, peritoneal fluid is increasingly produced, which is then referred to as ascites. The causes of ascites can therefore be manifold. One possible cause of ascites is the growth of a tumour in the abdominal and pelvic cavities. In patients with OC, tumour cells can be shed at a very early stage of the disease and spread within the abdominal and pelvic cavity via the peritoneal fluid to form metastases on the peritoneal wall, in the omentum and the serous membranes of other organs. Even though first-line therapy (surgery and chemotherapy) frequently achieves clinically tumour-free state, most patients relapse due to the outgrowth of residual cancer cells.

Markers (biomarkers) are substrates of the human or animal body or in human or animal body fluids such as blood, serum, plasma, or peritoneal fluid, which are useful for diagnosis of diseases and/or for determination of the prognosis of a disease. Markers are measurable indicators of the presence or severity of a disease. The concentration of markers is determined in order to obtain a diagnosis and/or prognosis. The concentration of markers can be increased or decreased, depending on the marker and the disease. Markers can be proteins, peptides, or oligopeptides; they also can be low molecular mass organic substances or elements.

### State of the Art

The following known methods can be used for the diagnosis of OC:
- Imaging diagnostics: Ultrasound devices, X-ray systems, magnetic resonance tomographs, computer tomographs or positron emission tomographs can be used for the diagnosis of OC, but are not suitable for the detection of early stages and small tumour masses. The small tumour masses in early stage OC are easily overlooked by imaging diagnostics, especially in peritoneal carcinomatosis or disseminated relapses. For example, a tumour mass smaller than 5 mm in diameter and peritoneal carcinomatosis are usually undetectable by imaging diagnostics. Furthermore, imaging diagnostics are expensive and the results depend on the experience and skill of the examiner. The use of X-rays poses a potential hazard for the patient, in particular for patients with an existing desire to have children. For these reasons, imaging diagnostics are not suitable for the screening for OC as a preventive medical check-up.
- Endoscopic examination: OC can be diagnosed by laparoscopic examination. However, laparoscopy is performed only in unclear symptomatic or otherwise suspicious situations, and is unsuitable for screening purposes or follow-up examinations after first-line therapy. In addition, laparoscopy is expensive and the results depend on the experience and skill of the examiner. Laparoscopy can have adverse sequelae for the patient because of the need to open the peritoneal (abdominal or pelvic) cavity. For these reasons, endoscopic examinations are not suitable for the screening of OC in patients undergoing a preventive medical check-up.
- Histological-pathological examination of tissue samples: This examination can only be carried out if tissue samples of the suspected tumour are available. Taking a tissue sample is an invasive procedure that can be associated with serious complications. Histological-pathological examinations are therefore unsuitable for screening or follow-up examinations or for preventive medical check-ups.
- Blood test: The general parameters such as cell count, differential blood count and clinical-chemical parameters are not meaningful with regard to the presence of early stage OC.
- Determination of the concentration of markers (biomarkers) like transthyretin (TTR), transferrin (TF), apolipoprotein A1 (APOA1), cancer antigen 125 (CA125, MUC16, mucin 16), human chorionic gonadotropin (HCG), alpha fetoprotein (AFP), lactate dehydrogenase (LDH), carcinoembryonic antigen (CEA), choriogonadotropin-beta (CGB), inhibin B, and human epididymis (epididymal) protein 4 (HE4, WFDC2) in blood plasma: This procedure is unreliable because false negative and/or false positive results often occur. Therefore, the determination of these markers cannot distinguish patients with OC from patients with benign diseases and healthy women with satisfactory certainty. For example, the most widely used marker for the detection of recurrent OC is CA125, but its routine clinical application has not resulted in any substantial improvement in patient survival, since it is neither a sensitive nor a highly specific OC marker. Nevertheless, most physicians use the biomarker CA125, so this marker is currently the gold standard. These markers, like many other biomarkers, are proteins, peptides, or oligopeptides. Their concentration in a biological sample can be measured by different methods. One method for example employs the SOMAscan® Proteomic Assay by SomaLogic Inc. The SOMAscan® Proteomic Assay currently measures up to approximately 1,300 markers or protein analytes in a biological sample for instance in serum, plasma, peritoneal fluid or cerebrospinal fluid. The SOMAscan® Proteomic Assay is able to quantify markers that span over eight logs in abundance from femtomolar to micromolar. The SOMAscan® Proteomic Assay uses aptamer technology. The employed aptamers (SOMAmer reagents) have a dual nature as protein affinity-binding reagents with defined three-dimensional structures, and unique nucleotide sequences recognizable by specific DNA hybridization probes. The SOMAscan® Proteomic Assay transforms the protein present in a biological sample into a specific DNA signal. The results of standard DNA quantification techniques are normalized to assure data consistency by correcting for variations due to the SOMAscan® Proteomic Assay, thereby allowing for comparison of samples across a plate. The normalized results obtained for replicate calibration samples included in the assay are then compared to a previous established reference calibration value. This generates a calibration scale factor that is applied to all samples. The final data are reported in relative fluorescent units (RFU) after normalization and calibration. A strong advantage of the SOMAscan® Proteomic Assay over mass spectrometry is its applicability to unfractionated blood plasma and related fluids like as peritoneal fluid in spite of the high abundance of blood proteins such as albumin, globulins, and fibrinogen, which has been a limiting factor for all previous mass spectrometry-based proteomic analysis of blood plasma.
- Multivariate index assay (MIA): A multivariate index assay for in vitro diagnosis is a method that combines the values of multiple markers using an interpretation function to yield a single, patient-specific result that is indented for use in the diagnosis of disease. For example, an 11-marker panel composed of CA125, CA19-9, EGF-R, C-reactive protein, myoglobin, apolipoprotein A1, apolipoprotein CIII, MIP-1alpha, IL-6, IL-18, and tenascin C was described for the diagnosis of OC. Although one of the currently available MIA shows a sensitivity of more than 91.3 % and a specificity around 98 %, this is not enough to achieve a positive predictive value of 10 % and a low number of false-positive results.
- Genetic tests of liquid biopsies: Some genetic alterations are associated with OC, for example a mutation in the TP53, BRCA1, or BRCA2 genes. However, genetic tests of liquid biopsies have not provided any tools for the detection of early stage OC.

For the diagnosis of recurrences of OC in the context of disease monitoring, determination of the concentration of CA125 in blood plasma is performed: Changes of CA125 in blood plasma concentration should indicate tumour growth or recurrence. However, the concentration of CA125 in blood plasma does not always increase even with proven recurrences, and may be increased in other conditions, such as endometriosis, ovarian cysts, pelvic inflammatory disease, uterine fibroids, leiomyomas, and menstruation. Therefore, both false negative and false positive results are common in this procedure. This is particularly disadvantageous, as in these cases no appropriate therapy is carried out despite existing primary OC or OC recurrences.

Scientists have been trying for several decades to overcome this inadequacy by combining CA125 with other markers, but these attempts all ultimately failed to diagnose OC at a stage where treatment can have an impact on disease outcome. A number of markers in blood, blood serum, or blood plasma has already been analysed in a non-systematic manner and suggested for the diagnosis of OC, but none of them was validated as a suitable marker for detection of early stage OC. Because of the low prevalence of OC, any screening method for diagnosing OC requires a minimum specificity and sensitivity of >99 % to achieve an acceptable positive predictive value. For the differential diagnosis of suspected or the detection of relapsed OC the requirements in terms of minimum specificity and sensitivity are lower to achieve an acceptable positive predictive value due to the much higher prior probability. For the diagnosis of suspected or relapsed OC the sensitivity is of particular relevance to avoid false negatives (unrecognized cancers or relapses). In this case a minimum specificity and sensitivity of >90 % would be desirable. None of the currently available methods fulfils this criterion (Nolen & Lokshin, 2012). Therefore, there are currently no suitable screening methods for the detection of early stage OC (early detection).

### Problems

There is currently no reliable screening method for the detection of early stage OC with a minimum specificity and sensitivity of >99 % to achieve an acceptable positive predictive value.

There is currently no reliable method for the early detection of relapsed OC.

This invention solves the following tasks:
- Improved diagnosis of OC;
- Improved diagnosis of recurrences of already treated OC;
- Improved diagnosis of early stages of OC;
- Improved screening of OC in individuals without any clinical signs.

A serious limitation of all previous efforts for diagnosing OC is the lack of any unbiased global analysis for the discovery of OC markers. Therefore, the inventors used this approach for the present invention.

### Solution

Claim 1 solves the inventive task. The main inventive steps are first the identification of the most significant markers in a biological sample (blood, blood plasma, or blood serum) that have the highest significance to the occurrence or recurrence of OC, and second the biostatistical analysis of the concentration of these markers in patients with OC in comparison with subjects with non-malignant diseases. This invention describes the markers for the diagnosis of OC, especially early stage and relapsed OC. This invention also describes the calculation of multi-marker scores and their use for identifying patients with OC.

The key feature of the invention is the selection of markers from a very large number of proteins, peptides, or oligopeptides in a biological sample (blood, blood plasma, blood serum, or other non-solid biological tissue) determined by an unbiased analysis that enables a reliable diagnosis of OC, especially early stage and relapsed OC. The inventors identified several markers out of 1,305 potential markers, which allow for a reliable diagnosis in patients with OC. In order to identify the most significant markers for OC the inventors measured (determinated) the concentration of 1,305 potential markers in blood plasma samples of 20 patients with proven OC (OC-plasma) and in blood plasma samples of 10 subjects with non-malignant diseases (N-plasma).

In order to perform the inventive method the individual (subject) must provide a biological sample (blood, blood plasma, or blood serum). These samples are obtained for example from patients with suspected OC, especially early stage and relapsed OC. These samples are also obtained from any healthy individual for screening purposes, in particular individuals with a family history of breast cancer or OC. Usually these samples are provided by a physician. The collection of blood in order to obtain a biological sample is not stressful for the patient and is a routine medical procedure. The most useful biological sample to apply the invention is blood plasma.

The inventive method for diagnosing ovarian carcinoma (OC) in a biological sample from an individual with suspected OC or suspected relapsed OC, comprises the following steps:
(i) measuring the concentration of at least the markers ASGR1, EPHA2, and SPON1 in the biological sample of said individual;
(ii) comparing the measured concentrations from step (i) with predefined thresholds;
(iii) assigning a score of 1 for each marker which measured concentration is above its predefined threshold;
(iv) assigning a score of 0 for each marker which measured concentration is below its predefined threshold;
(v) adding the scores from step (iii) and (iv) to form a multi-marker score;
(vi) dividing the multi-marker score from step (v) by the total number of measured markers from step (i);
(vii) multiplying the result of step (vi) by 100 to obtain a percentage;
(viii) comparing this percentage to whether it is ≥60%;

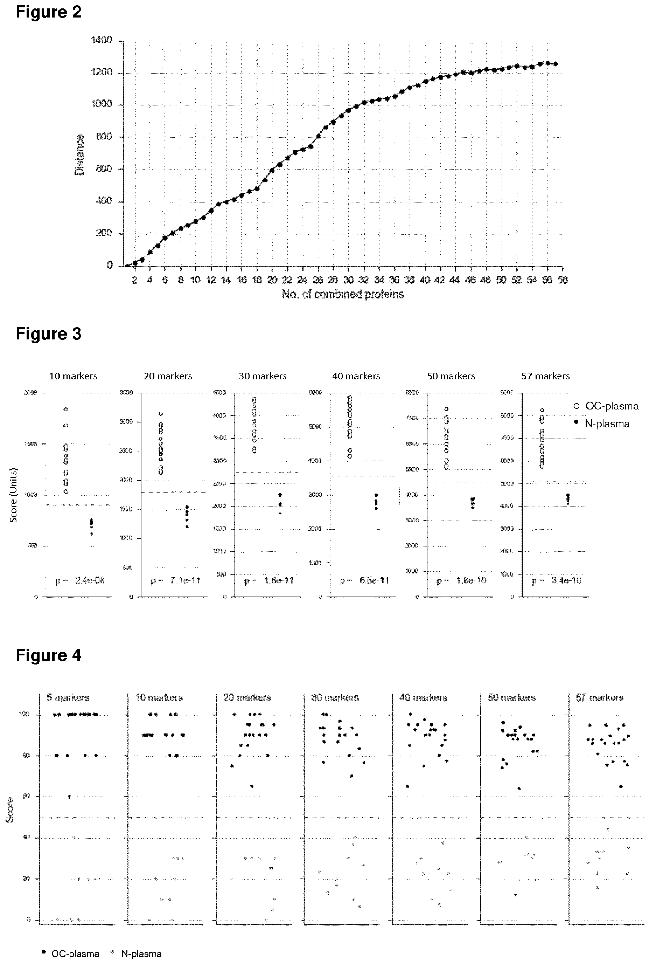

If the percentage of step (vii) is higher or equal than 60% the said individual has OC. If the percentage of step (vii) is lower than 60% the said individual has no OC. The predefined thresholds according to step (ii) for each marker which concentration is measured by SOMAscan® Proteomic Assay can be seen in Table 2, most right column (Threshold for Figure 4 (Units)). If the concentration of the markers is measured by another method, like as ELISA, other antibody-based techniques, microarray-based technology, other target-proteomics-based methods or mass spectrometry the thresholds for each marker must been adjusted.

In one embodiment of the invention the first step of the inventive method is the determination of the concentration of the markers ASGR1, SPON1, FUT5, EPHB2, SST, EPHA2, ANGPT2, C5a, EFNA4, SERPING1, PYY, CCL14, IL18BP, SPP1, SECTM1, TFPI, IGFBP2, FTH1, TNFRSF21, COLEC12, PRSS22, CCDC80, PDGFRA, HAMP, EFNA5, ENO2, STC1, SUMO2, COTL1, TNFSF15, HTRA2, VEGF, LBP, NRP1, C2, FABP3, RELT, FLRT2, C9, alpha-1-Antichymotrypsin, FSTL1, CST3, THBS2, TNFRSF8, SCARF1, B2M, Coagulation Factor IXab, GAS1, HSP90AB1, RBM39, NID1, ENO1, CRP, HSP90AA1, FSHB, and VWF in a biological sample obtained from a subject. Table 2 lists these markers with annotations. The concentration of marker TIMP1 is very low and close to background, therefore TMP1 is no suitable marker. In order to determine the concentration of these markers in a biological sample any suitable method can be applied, e.g. ELISA-based techniques, mass spectrometry, antibody- or aptamer-based microarrays, or any other suitable quantification method, however preferably a SOMAscan® Proteomic Assay.

In a second step, the inventive method for diagnosing OC comprises the calculation of multi-marker scores. The concentrations of at least three markers listed in Table 2 in conjunction with suitable biostatistical analyses are used to predict the presence of OC in an individual. The multi-marker score is calculated as the percentage of markers the concentration of which is higher than a predefined threshold. The threshold is defined as the percentile (lower tercile) reflecting the percentage of N-plasma samples in a group of N- and OC-plasma samples. For example, 10 out of 30 is the 33^{th} percentile for 20 OC-plasma and 10 N-plasma samples. The corresponding values of the concentrations of these markers are listed in Table 2 (third column, Distance). If the percentage of markers that are higher than the threshold is 60% or higher the suspected diagnosis for this individual is OC and further examinations are appropriate. If the percentage of markers that are higher than the threshold is lower than 40% there is no suspicion of OC for this individual and no further examinations are necessary.

In an embodiment, the concentrations of at least the markers ASGR1, EPHA2, SPON1, and THBS2 are determined in step (i) in the biological sample of an individual.

In an embodiment, the concentrations of at least the markers ASGR1, EPHA2, SPON1, THBS2, and FUT5 are determined in step (i) in the biological sample of an individual.

In an embodiment, the concentrations of at least the markers ASGR1, EPHA2, SPON1, THBS2, FUT5, EFNA4, CCDC80, FTH1, and C5a are determined in step (i) in the biological sample of an individual.

The concentration of these markers can be measured by ELISA-based techniques, mass spectrometry, antibody- or aptamer-based microarrays or any other suitable quantification method, however preferably a SOMAscan® Proteomic Assay. According to the method of measuring the concentrations the predefined thresholds of each marker must been adjusted.

In order to execute the method according to the invention a device (e.g. test kit) can be used. The device for diagnosing ovarian carcinoma (OC) in a biological sample from an individual according to claim 9 comprises at least following components:
- a measuring unit for measuring the concentration of at least the markers ASGR1, EPHA2, and SPON1 in the biological sample of said individual whereby this unit is capable to forward the measured concentrations to a data processing unit;
- a data processing unit which receives the measured concentrations from the measuring unit and which executes the steps (ii) to (viii) of claim 1 and forwards the results to the output unit;
- an input unit for data entry which is connectable to a database for the input of at least the predefined thresholds and which forwards data to the data processing unit;
- an output unit for data output which indicates OC, if the percentage is higher or equal than 60% and where the individual has no OC, if the percentage is lower than 60%.

In an embodiment the device for diagnosing OC calculates the multi-marker scores by counting the number of markers which concentration is higher than the threshold. The threshold is defined as the 33^{th} percentile (lower tercile) of all 30 plasma samples (20 OC-plasma and 10 N-plasma) as listed in the right-most column of Table 2 (Threshold for Figure 4 in units). If the percentage of markers that are higher than the threshold is 60% or higher the suspected diagnosis for this individual is OC and further examinations are appropriate. If the percentage of markers that are higher than the threshold is lower than 40% there is no suspicion of OC for this individual and no further examinations are necessary.

In an embodiment this device is a test kit for measuring the concentration of the following markers in a biological sample like as blood, blood plasma, or blood serum from a subject with suspected OC or with suspected recurrence of OC: ASGR1, SPON1, FUT5, EPHB2, SST, EPHA2, ANGPT2, C5a, EFNA4, SERPING1, PYY, CCL14, IL18BP, SPP1, SECTM1, TFPI, IGFBP2, FTH1, TNFRSF21, COLEC12, PRSS22, CCDC80, PDGFRA, HAMP, EFNA5, ENO2, STC1, SUMO2, COTL1, TNFSF15, HTRA2, VEGF, LBP, NRP1, C2, FABP3, RELT, FLRT2, C9, alpha-1-Antichymotrypsin, FSTL1, CST3, THBS2, TNFRSF8, SCARF1, B2M, Coagulation Factor IXab, GAS1, HSP90AB1, RBM39, NID1, ENO1, CRP, HSP90AA1, FSHB, and VWF.

The invention also comprises a computer software to perform the method according to claim 1. In a preferred embodiment, this computer software is implemented in the data processing unit and performs the steps (ii) to (viii) of claim 1.

### Embodiments

### 1. Determination of markers in biological samples like as blood, blood plasma, or blood serum as markers for OC

Extensive investigations and extensive biostatistical analyses of the inventors have shown that there is a high correlation between clinical findings and the concentration of certain markers in blood, blood plasma or blood serum of patients with OC. Since most markers of interest in OC-patients are presumably proteins, a targeted-proteomics-based global analysis of blood plasma was performed in patients with proven OC (OC-patients, OC-plasma) and in subjects with non-malignant diseases (N-patients, N-plasma). Subjects with non-malignant diseases had ovarian cysts or myoma uteri. In order to perform this proteome analysis the aptamer-based SOMAscan® Proteomic Assay was utilized. The data from the proteome analysis were then biostatistically evaluated.

For proteome analysis, 20 samples of blood plasma were examined from patients with high-grade serous OC (OC-plasma). For comparison, 10 samples of blood plasma from subjects with non-malignant diseases were examined (N-plasma). The concentration of 1,305 markers was measured in these samples.

The inventive step is to select from these 1,305 markers in the blood plasma those combinations that have a highly significant correlation with the presence of OC. Biostatistical analyses identified significantly higher concentrations in OC-plasma versus N-plasma for 57 marker proteins (see Table 1). Table 1 shows that some markers are present in OC-plasma at significantly higher levels than in N-plasma (see fold change). The indicated p-values were determined by two-sided unpaired t-test and adjusted for multiple hypothesis testing by Benjamini-Hochberg correction. The rank order reflects the increasing adjusted p-value.

**Table 1**

| **No**. | **Marker** | **Fold change (OC- vs. N-plasma)** | **p-value** | **adjusted p-value** | | **No.** | **Marker** | **Fold change (OC- vs. N-plasma)** | **p-value** | **adjusted p-value** |
|---|---|---|---|---|---|---|---|---|---|---|
| **1** | IGFBP2 | 4.63 | 0.000001 | 0.0003 | | **85** | RARRES2 | 1.20 | 0.009726 | 0.0783 |
| **2** | TIMP1 | 1.50 | 0.000001 | 0.0003 | | **86** | SHBG | 1.57 | 0.009941 | 0.0796 |
| **3** | C9 | 1.59 | 0.000008 | 0.0012 | | **87** | TNFRSF1B | 1.42 | 0.010329 | 0.0821 |
| **4** | THBS2 | 2.75 | 0.000014 | 0.0016 | | **88** | DCTPP1 | 1.73 | 0.010507 | 0.0821 |
| **5** | NRP1 | 1.58 | 0.000020 | 0.0019 | | **89** | REG1A | 1.83 | 0.010420 | 0.0821 |
| **6** | SPP1 | 2.93 | 0.000054 | 0.0032 | | **90** | EPHB6 | 1.24 | 0.010458 | 0.0821 |
| **7** | TNFRSF8 | 1.79 | 0.000073 | 0.0040 | | **91** | CAPG | 2.69 | 0.011329 | 0.0875 |
| **8** | PDGFRA | 1.72 | 0.000103 | 0.0052 | | **92** | HAPLN1 | 1.30 | 0.011424 | 0.0877 |
| **9** | C5a | 1.50 | 0.000233 | 0.0095 | | **93** | ESAM | 1.19 | 0.011682 | 0.0885 |
| **10** | GAS1 | 1.38 | 0.000287 | 0.0104 | | **94** | TNFRSF1A | 1.61 | 0.011929 | 0.0885 |
| **11** | TFPI | 1.39 | 0.000309 | 0.0106 | | **95** | SEMA6B | 1.42 | 0.011900 | 0.0885 |
| **12** | FSTL1 | 1.22 | 0.000305 | 0.0106 | | **96** | IL13RA1 | 1.30 | 0.012134 | 0.0890 |
| **13** | EPHA2 | 1.83 | 0.000317 | 0.0106 | | **97** | BCL2L2 | 1.19 | 0.012281 | 0.0890 |
| **14** | CRP | 3.57 | 0.000350 | 0.0112 | | **98** | 14-3-3 | 1.19 | 0.013353 | 0.0932 |
| **15** | PRSS22 | 2.50 | 0.000377 | 0.0114 | | **99** | ESM1 | 1.29 | 0.013333 | 0.0932 |
| **16** | RELT | 1.58 | 0.000376 | 0.0114 | | **100** | GPI | 1.56 | 0.013748 | 0.0954 |
| **17** | SCARF1 | 1.34 | 0.000411 | 0.0120 | | **101** | BID | 1.23 | 0.014368 | 0.0978 |
| **18** | COTL1 | 1.18 | 0.000414 | 0.0120 | | **102** | TLR2 | 1.14 | 0.014660 | 0.0984 |
| **19** | RBM39 | 2.19 | 0.000422 | 0.0120 | | **103** | DDR2 | 1.15 | 0.014582 | 0.0984 |
| **20** | LBP | 1.39 | 0.000433 | 0.0120 | | **104** | CXCL3 | 1.47 | 0.015040 | 0.0996 |
| **21** | EFNA4 | 1.38 | 0.000455 | 0.0121 | | **105** | KLK11 | 3.68 | 0.015432 | 0.1011 |
| **22** | ASGR1 | 1.61 | 0.000471 | 0.0123 | | **106** | CXCL16 | 1.27 | 0.015650 | 0.1015 |
| **23** | FTH1 | 4.99 | 0.000487 | 0.0123 | | **107** | CTSD | 1.47 | 0.015750 | 0.1015 |
| **24** | FLRT2 | 2.15 | 0.000581 | 0.0138 | | **108** | N-terminal pro-BNP | 2.77 | 0.016211 | 0.1034 |
| **25** | EPHB2 | 1.69 | 0.000574 | 0.0138 | | **109** | alpha-1-antichymotrypsin complex | 1.81 | 0.016239 | 0.1034 |
| **26** | Coagulation Factor IXab | 1.33 | 0.000717 | 0.0153 | | **110** | TNF | 1.22 | 0.016367 | 0.1037 |
| **27** | ANGPT2 | 1.43 | 0.000698 | 0.0153 | | **111** | THPO | 1.19 | 0.016532 | 0.1042 |
| **28** | a1-Antichymotrypsin | 1.39 | 0.000729 | 0.0153 | | **112** | PTN | 1.32 | 0.016669 | 0.1046 |
| **29** | HAMP | 4.78 | 0.000780 | 0.0161 | | **113** | CX3CL1 | 1.33 | 0.017231 | 0.1061 |
| **30** | ENO2 | 1.80 | 0.001017 | 0.0192 | | **114** | SELP | 1.18 | 0.017214 | 0.1061 |
| **31** | VEGF | 1.64 | 0.001118 | 0.0209 | | **115** | XRCC6 | 1.35 | 0.017663 | 0.1071 |
| **32** | SUMO2 | 1.51 | 0.001212 | 0.0217 | | **116** | FLRT3 | 1.45 | 0.017731 | 0.1071 |
| **33** | FUT5 | 2.20 | 0.001211 | 0.0217 | | **117** | IL2 | 1.17 | 0.017949 | 0.1079 |
| **34** | VWF | 1.66 | 0.001346 | 0.0231 | | **118** | ADAMTS15 | 1.13 | 0.018123 | 0.1085 |
| **35** | IL18BP | 1.37 | 0.001321 | 0.0231 | | **119** | NUDCD3 | 1.31 | 0.018211 | 0.1085 |
| **36** | CCDC80 | 1.67 | 0.001361 | 0.0231 | | **120** | IBSP | 1.27 | 0.018967 | 0.1125 |
| **37** | SECTM1 | 1.43 | 0.001490 | 0.0241 | | **121** | F3 | 1.51 | 0.019196 | 0.1128 |
| **38** | HSP90AB1 | 1.57 | 0.001498 | 0.0241 | | **122** | APCS | 1.18 | 0.019570 | 0.1143 |
| **39** | HSP90AA1 | 1.62 | 0.001647 | 0.0259 | | **123** | IL5RA | 1.56 | 0.019886 | 0.1143 |
| **40** | CCL14 | 1.21 | 0.001689 | 0.0259 | | **124** | HSPA1A | 1.22 | 0.019740 | 0.1143 |
| **41** | COLEC12 | 1.69 | 0.001932 | 0.0280 | | **125** | C7 | 1.27 | 0.019848 | 0.1143 |
| **42** | TNFRSF21 | 1.33 | 0.002083 | 0.0295 | | **126** | EFEMP1 | 1.28 | 0.020093 | 0.1150 |
| **43** | TNFSF15 | 1.54 | 0.002237 | 0.0311 | | **127** | SHC1 | 1.50 | 0.020320 | 0.1153 |
| **44** | B2M | 1.59 | 0.002341 | 0.0316 | | **128** | MMP7 | 1.46 | 0.020560 | 0.1153 |
| **45** | EFNA5 | 1.37 | 0.002341 | 0.0316 | | **129** | BOC | 1.35 | 0.021832 | 0.1207 |
| **46** | STC1 | 1.67 | 0.002401 | 0.0320 | | **130** | MMP13 | 1.24 | 0.022883 | 0.1246 |
| **47** | SPON1 | 2.24 | 0.002852 | 0.0365 | | **131** | PIAS4 | 1.24 | 0.022912 | 0.1246 |
| **48** | SERPING1 | 1.35 | 0.003031 | 0.0384 | | **132** | IGFBP1 | 1.69 | 0.022850 | 0.1246 |
| **49** | FABP3 | 1.83 | 0.003151 | 0.0395 | | **133** | UNC5C | 1.29 | 0.023147 | 0.1248 |
| **50** | NID1 | 1.25 | 0.003199 | 0.0398 | | **134** | NAMPT | 1.30 | 0.023579 | 0.1254 |
| **51** | ENO1 | 1.75 | 0.003384 | 0.0409 | | **135** | CAMK1 | 1.22 | 0.023683 | 0.1254 |
| **52** | HTRA2 | 1.25 | 0.003431 | 0.0411 | | **136** | TNFRSF4 | 1.33 | 0.023401 | 0.1254 |
| **53** | CST3 | 1.38 | 0.003630 | 0.0431 | | **137** | GP6 | 1.44 | 0.024628 | 0.1277 |
| **54** | FSHB | 4.30 | 0.003848 | 0.0448 | | **138** | HAVCR2 | 1.50 | 0.024713 | 0.1277 |
| **55** | C2 | 1.18 | 0.003848 | 0.0448 | | **139** | CSF1 | 1.18 | 0.025068 | 0.1288 |
| **56** | PYY | 1.72 | 0.003981 | 0.0460 | | **140** | BMP10 | 1.22 | 0.025854 | 0.1323 |
| **57** | SST | 1.28 | 0.004203 | 0.0477 | | **141** | TGFB3 | 1.19 | 0.026232 | 0.1337 |
| **58** | GDF15 | 2.35 | 0.004504 | 0.0502 | | **142** | GCG | 1.59 | 0.026562 | 0.1348 |
| **59** | MRC1 | 1.42 | 0.004654 | 0.0515 | | **143** | APP | 1.24 | 0.027532 | 0.1377 |
| **60** | IFNG | 1.19 | 0.004838 | 0.0524 | | **144** | TNFRSF9 | 1.23 | 0.032588 | 0.1564 |
| **61** | SNRPF | 1.15 | 0.005208 | 0.0548 | | **145** | C5b, 6 Complex | 1.22 | 0.032813 | 0.1569 |
| **62** | Coagulation Factor IX | 1.29 | 0.005195 | 0.0548 | | **146** | EIF4G2 | 1.30 | 0.033432 | 0.1586 |
| **63** | S100A12 | 2.02 | 0.005295 | 0.0549 | | **147** | STAT3 | 1.23 | 0.035224 | 0.1659 |
| **64** | EFNB1 | 1.22 | 0.005494 | 0.0560 | | **148** | IL1 RL1 | 1.40 | 0.035614 | 0.1672 |
| **65** | CD59 | 1.20 | 0.005538 | 0.0560 | | **149** | ITGA1 | 1.22 | 0.037340 | 0.1740 |
| **66** | MIF | 1.25 | 0.005688 | 0.0571 | | **150** | FGG | 1.14 | 0.037638 | 0.1748 |
| **67** | ICAM1 | 1.20 | 0.005813 | 0.0575 | | **151** | SLITRK5 | 1.18 | 0.037790 | 0.1749 |
| **68** | MBL2 | 1.98 | 0.005897 | 0.0579 | | **152** | EFNB2 | 1.25 | 0.037975 | 0.1751 |
| **69** | CSRP3 | 1.41 | 0.006081 | 0.0588 | | **153** | CST1 | 1.56 | 0.038334 | 0.1755 |
| **70** | IL15RA | 1.32 | 0.006188 | 0.0589 | | **154** | FAS | 1.19 | 0.039922 | 0.1809 |
| **71** | CFHR5 | 1.46 | 0.006147 | 0.0589 | | **155** | CHGA | 1.75 | 0.040383 | 0.1824 |
| **72** | C5 | 1.31 | 0.006732 | 0.0628 | | **156** | CRK | 1.34 | 0.040650 | 0.1829 |
| **73** | SIRPA | 1.61 | 0.006789 | 0.0628 | | **157** | LAYN | 1.36 | 0.041131 | 0.1842 |
| **74** | DSC2 | 1.42 | 0.006987 | 0.0638 | | **158** | ADAM12 | 1.60 | 0.043100 | 0.1900 |
| **75** | COL18A1 | 1.43 | 0.007203 | 0.0653 | | **159** | Ck-b-8-1 | 1.51 | 0.044025 | 0.1921 |
| **76** | COL18A1 SPINT2 | 1.41 | 0.007537 | 0.0674 | | **160** | CXCL8 | 1.56 | 0.044760 | . 0.1941 |
| **77** | FSTL3 | 1.39 | 0.007779 | 0.0681 | | **161** | PARK7 | 1.18 | 0.045861 | 0.1975 |
| **78** | PLAUR | 1.47 | 0.007910 | 0.0684 | | **162** | BCAM | 1.85 | 0.046632 | 0.2002 |
| **79** | EFNA2 | 1.30 | 0.007859 | 0.0684 | | **163** | FIGF | 1.24 | 0.048191 | 0.2055 |
| **80** | ACP5 | 1.30 | 0.008891 | 0.0734 | | **164** | TFF1 | 1.44 | 0.048982 | 0.2075 |
| **81** | GSTP1 | 1.39 | 0.008835 | 0.0734 | | **165** | TNFSF13B | 1.31 | 0.049333 | 0.2077 |
| **82** | WISP3 | 1.15 | 0.009044 | 0.0742 | | **166** | MMP3 | 1.22 | 0.049265 | 0.2077 |
| **83** | LUM | 1.40 | 0.009184 | 0.0749 | | **167** | TEK | 1.17 | 0.049513 | 0.2078 |
| **84** | VEGF121 | 1.66 | 0.009390 | 0.0761 | | | | | | |

### 2. Biostatistical evaluation of proteome analysis and calculation of multi-marker scores

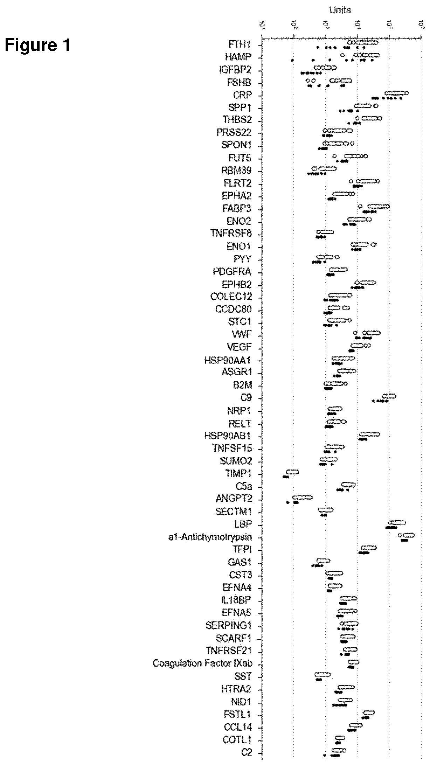

In a first step, all markers (n=167) with higher median levels in OC-plasma compared to N-plasma (p-value <0.05 by two-sided unpaired t-test; Table 1) were identified. Of these, 57 markers were significant after adjustment for multiple hypothesis testing by Benjamini-Hochberg correction (Table 1). Figure 1 is a graphical representation of the data for these 57 upregulated markers, which shows that none of the markers on its own is able to achieve a complete separation of all OC-plasma and all N-plasma samples.

Within the set of significantly upregulated markers, the marker with the maximum distance between the minimum of all OC-plasma and the maximum of all N-plasma was identified. This marker is TIMP1 (Table 2).

A marker-wise normalization of SOMAscan® Proteomic Assay units was performed and the marker in the set of significantly upregulated markers that maximally increases the distance after adding up marker-wise normalized SOMAscan® Proteomic Assay units was identified. This was repeated successively for each of the significantly upregulated markers to obtain combinations of successively increasing size (Table 2, Figure 2). An increase in distance was found with combinations of up to approximately 40 markers, which levelled off thereafter (Figure 2).

Table 2 shows the markers that are used for the clear distinction between patients with OC (OC-plasma) and subjects with non-malignant diseases (N-plasma). "Distance" indicates the difference of the multi-marker scores between OC-plasma and N-plasma after the measured concentrations were normalized and added up using the indicated reference values.

The numbers (No.) refer to the rank of the respective marker in the combinations in Figure 2 (e.g., the combination of 10 markers contains marker 1-10 listed in Table 2). The most right column (Threshold for Figure 4) shows the reference (median of marker concentrations in OC-plasma) used for marker-wise normalization of measured concentrations (measured value / reference * 100).

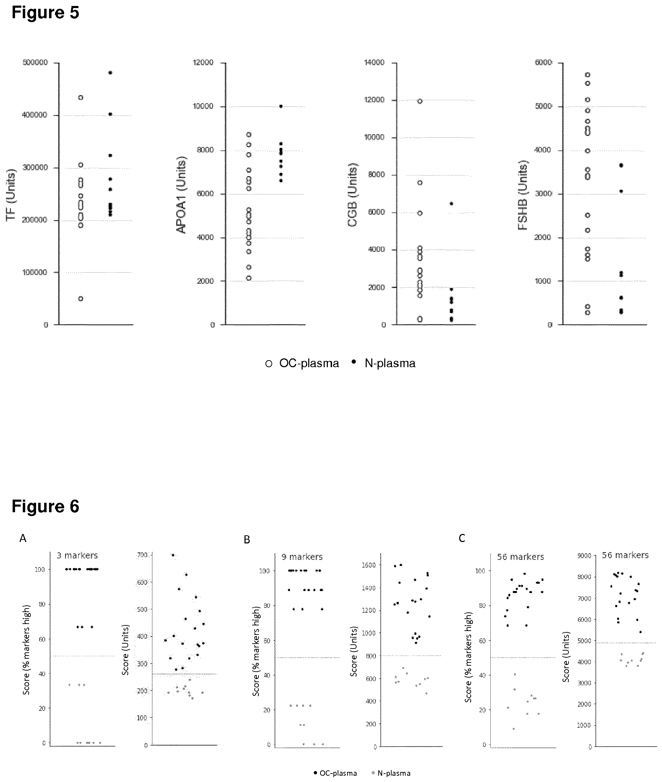

The complete separation achieved by the inventive method is demonstrated in Figure 3 for the set of the top 10, 20, 30, 40, 50, and 57 markers in Table 2. In contrast, other markers claimed in the state of the art as markers for OC, such as apolipoprotein A1 (APOA1), transferrin (TF), follicle-stimulating hormone-beta (FSHB), and choriogonadotropin-beta (CGB) were not suitable to separate OC-plasma and N-plasma samples in the cohort of patients shown in Figure 5.

**Table 2**

| **No.** | **Marker (gene symbol)** | **Distance** | **Description** | **Reference value for normalisation for** **Fig. 2, 3** **(Units)** | **Threshold for** **Figure 4** **(Units)** |
|---|---|---|---|---|---|
| 1 | TIMP1 | 1 | TIMP metallopeptidase inhibitor 1 | 82 | 64 |
| 2 | ASGR1 | 19 | asialoglycoprotein receptor 1 | 3806 | 2873 |
| 3 | SPON1 | 40 | spondin 1, extracellular matrix protein | 1845 | 1025 |
| 4 | FUT5 | 85 | fucosyltransferase 5 (alpha (1,3) fucosyltransferase) | 7772 | 4395 |
| 5 | EPHB2 | 125 | EPH receptor B2 | 17745 | 11352 |
| 6 | SST | 173 | somatostatin | 766 | 635 |
| 7 | EPHA2 | 204 | EPH receptor A2 | 2728 | 1896 |
| 8 | ANGPT2 | 232 | angiopoietin 2 | 156 | 125 |
| 9 | C5a | 251 | complement 5a | 4469 | 3775 |
| 10 | EFNA4 | 274 | ephrin-A4 | 1747 | 1356 |
| 11 | SERPING1 | 301 | serpin peptidase inhibitor, clade G, member 1 | 6296 | 4899 |
| 12 | PYY | 344 | peptide YY | 1044 | 665 |
| 13 | CCL14 | 385 | chemokine (C-C motif) ligand 14 | 7981 | 7128 |
| 14 | IL18BP | 400 | interleukin 18 binding protein | 4543 | 3770 |
| 15 | SPP1 | 414 | secreted phosphoprotein 1 | 17827 | 9584 |
| 16 | SECTM1 | 439 | secreted and transmembrane 1 | 1082 | 793 |
| 17 | TFPI | 460 | tissue factor pathway inhibitor | 22788 | 18007 |
| 18 | IGFBP2 | 478 | insulin-like growth factor binding protein 2, 36kDa | 1248 | 522 |
| 19 | FTH1 | 534 | ferritin, heavy polypeptide 1 | 14393 | 5726 |
| 20 | TNFRSF21 | 594 | TNF receptor superfamily, member 21 | 6397 | 4950 |
| 21 | COLEC12 | 633 | collectin sub-family member 12 | 2835 | 1774 |
| 22 | PRSS22 | 670 | protease, serine, 22 | 2608 | 1297 |
| 23 | CCDC80 | 706 | coiled-coil domain containing 80 | 1963 | 1392 |
| 24 | PDGFRA | 724 | platelet-derived GF receptor, alpha polypeptide | 2266 | 1520 |
| 25 | HAMP | 745 | hepcidin antimicrobial peptide | 26749 | 12420 |
| 26 | EFNA5 | 806 | ephrin-A5 | 3944 | 3023 |
| 27 | ENO2 | 861 | enolase 2 (gamma, neuronal) | 10720 | 6851 |
| 28 | STC1 | 895 | stanniocalcin 1 | 2058 | 1491 |
| 29 | SUMO2 | 935 | small ubiquitin-like modifier 2 | 1354 | 963 |
| 30 | COTL1 | 966 | coactosin-like 1 (Dictyostelium) | 2800 | 2547 |
| 31 | TNFSF15 | 990 | TNF (ligand) superfamily, member 15 | 1841 | 1241 |
| 32 | HTRA2 | 1016 | HtrA serine peptidase 2 | 3302 | 2844 |
| 33 | VEGF | 1024 | vascular endithelial GF (VEGF-121 + VEGF-C) | 10156 | 7093 |
| 34 | LBP | 1033 | lipopolysaccharide binding protein | 176603 | 138478 |
| 35 | NRP1 | 1041 | neuropilin 1 | 2169 | 1708 |
| 36 | C2 | 1055 | complement component 2 | 2378 | 2080 |
| 37 | FABP3 | 1082 | fatty acid binding protein 3, muscle and heart | 35339 | 27383 |
| 38 | RELT | 1110 | RELT tumor necrosis factor receptor | 2053 | 1396 |
| 39 | FLRT2 | 1124 | fibronectin leucine rich transmembrane protein 2 | 20650 | 12169 |
| 40 | C9 | 1146 | complement component 9 | 97417 | 76859 |
| 41 | SERPINA3 | 1160 | alpha-1-Antichymotrypsin | 419775 | 313890 |
| 42 | FSTL1 | 1170 | follistatin-like 1 | 22074 | 18470 |
| 43 | CST3 | 1182 | cystatin C | 1903 | 1465 |
| 44 | ADAMTS13 | 1190 | ADAM metallopeptidase with thrombospondin type 1 motif, 13 | 2555 | 2518 |
| 45 | F9ab | 1205 | coagulation Factor IXab | 7565 | 6293 |
| 46 | THBS2 | 1199 | thrombospondin 2 | 23452 | 11275 |
| 47 | NOTCH1 | 1216 | notch 1 | 13831 | 13938 |
| 48 | TNFRSF8 | 1222 | TNF receptor superfamily, member 8 | 1027 | 747 |
| 49 | AHSG | 1219 | alpha-2-HS-glycoprotein | 1027 | 1032 |
| 50 | B2M | 1225 | beta-2-microg lobulin | 1977 | 1326 |
| 51 | HS6ST1 | 1236 | heparan sulfate 6-O-sulfotransferase 1 | 895 | 895 |
| 52 | SCARF1 | 1243 | scavenger receptor class F, member 1 | 4895 | 4122 |
| 53 | HSP90AB1 | 1236 | heat shock protein 90kDa alpha, class B member 1 | 22499 | 14890 |
| 54 | MAP2K4 | 1237 | mitogen-activated protein kinase kinase 4 | 18865 | 17777 |
| 55 | ENO1 | 1260 | enolase 1, (alpha) | 14777 | 9933 |
| 56 | GAS1 | 1265 | growth arrest-specific 1 | 757 | 609 |
| 57 | RBM39 | 1256 | RNA binding motif protein 39 | 1063 | 638 |

### 3. Markers suitable for diagnosing OC

The invention shows that even a combination of fewer markers, e.g., the top 5 markers listed in Table 2 enables a complete separation of OC-plasma samples from N-plasma samples, which slightly increases when more markers are combined. For diagnostic applications, it is essential to include only markers in such combinations. The inventors therefore excluded markers that are present at very low levels (i.e., close to background) and can therefore not be accurately determined. This applies to marker TIMP1. This combination of the following 56 markers achieves a specificity and sensitivity of 100% for the cohort of 30 patients (Figure 6C): ASGR1, SPON1, FUT5, EPHB2, SST, EPHA2, ANGPT2, C5a, EFNA4, SERPING1, PYY, CCL14, IL18BP, SPP1, SECTM1, TFPI, IGFBP2, FTH1, TNFRSF21, COLEC12, PRSS22, CCDC80, PDGFRA, HAMP, EFNA5, ENO2, STC1, SUMO2, COTL1, TNFSF15, HTRA2, VEGF, LBP, NRP1, C2, FABP3, RELT, FLRT2, C9, alpha-1-Antichymotrypsin, FSTL1, CST3, THBS2, TNFRSF8, SCARF1, B2M, Coagulation Factor IXab, GAS1, HSP90AB1, RBM39, NID1, ENO1, CRP, HSP90AA1, FSHB, and VWF.

The inventors also performed bootstrapping analyses by simulation of data sets with added noise to the measured values. The most robust markers identified by this approach are ASGR1, CCDC80, EFNA4, EPHA2, IGFBP2, SPON1, SPP1, and THBS2. These eight markers were retested in all possible combinations by the approaches described above (as in Figures 2 to 4), which identified ASGR1, EPHA2 and SPON1 as the most crucial markers for an optimal discrimination of OC-plasma samples and N-plasma samples. Therefore, the inventors refer to this combination as the core signature.

The inventors then tested which of the markers in Table 1 (except TIPMP1), when added to this core signature, were able to improve the discrimination of OC-plasma and N-plasma samples further (Figure 6). These were THBS2, FUT5, EFNA4, CCDC80, FTH1, C5a, and SPP1, yielding the following suitable combinations:
Combination of three markers: ASGR1, EPHA2, SPON1
Combination of four markers: ASGR1, EPHA2, SPON1, THBS2
Combination of five markers: ASGR1, EPHA2, SPON1, THBS2, FUT5
Combination of six markers: ASGR1, EPHA2, SPON1, THBS2, FUT5, EFNA4
Combination of seven markers: ASGR1, EPHA2, SPON1, THBS2, FUT5, EFNA4, CCDC80
Combination of eight markers: ASGR1, EPHA2, SPON1, THBS2, FUT5, EFNA4, CCDC80, FTH1
Combination of nine markers: ASGR1, EPHA2, SPON1, THBS2, FUT5, EFNA4, CCDC80, FTH1, C5a
Combination of ten markers: ASGR1, EPHA2, SPON1, THBS2, FUT5, EFNA4, CCDC80, FTH1, C5a, SPP1

All combinations listed above achieve a specificity and sensitivity of 100% for the cohort of 30 patients (OC-patients and N-patients). Even if only the three-marker combination ASGR1, EPHA2, and SPON1 (Figure 6A) or the nine-marker combination ASGR1, EPHA2, SPON1, THBS2, FUT5, EFNA4, CCDC80, FTH1, and C5a (Figure 6B) is used for separating OC-plasma and N-plasma samples a better specificity and sensitivity is achieved than in the state of the art.

For a clear distinction between patients with OC and patients without malignant diseases, the following markers from Table 2 can be included in marker combinations: ASGR1, SPON1, FUT5, EPHB2, SST, EPHA2, ANGPT2, C5a, EFNA4, SERPING1, PYY, CCL14, IL18BP, SPP1, SECTM1, TFPI, IGFBP2, FTH1, TNFRSF21, COLEC12, PRSS22, CCDC80, PDGFRA, HAMP, EFNA5, ENO2, STC1, SUMO2, COTL1, TNFSF15, HTRA2, VEGF, LBP, NRP1, C2, FABP3, RELT, FLRT2, C9, alpha-1-Antichymotrypsin, FSTL1, CST3, THBS2, TNFRSF8, SCARF1, B2M, Coagulation Factor IXab, GAS1, HSP90AB1, RBM39, NID1, ENO1, CRP, HSP90AA1, FSHB, and VWF. These markers can be used in suitable combinations of approximately 3-30 markers in a test kit for the differentiation between patients with OC and subjects with non-malignant diseases.

A preferred combination is comprised of at least the nine markers ASGR1, EPHA2, SPON1, THBS2, FUT5, EFNA4, CCDC80, FTH1, and C5a (Figure 6B). These combinations of markers allow the early detection of OC or the detection of relapsed OC in an individual.

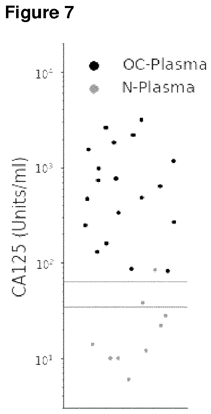

Multi-marker scores are calculated by counting the number of markers which concentration is higher than the threshold, predefined as the percentile that equals the percentage of plasma samples from patients with non-malignant disease within a group of plasma samples from patients with OC and plasma samples from patients with non-malignant disease. In this case, the threshold is defined as the 33^{th} percentile of all 30 plasma samples (20 OC-plasma and 10 N-plasma) as listed in the right-most column of Table 2 (Threshold for Figure 4 in units). If the percentage of markers that are higher than the threshold is 60% or higher the suspected diagnosis for this individual is OC. If the percentage of markers that are higher than the threshold is lower than 40% there is no suspicion of OC for this individual. The inventors compared the results of the inventive method with the results of measuring the concentration of the biomarker CA125 in the OC-plasma samples and the N-plasma samples by a clinically approved ELISA, which is the gold standard at time. The results are shown in Figure 7. It is obvious that the inventive method achieves much better results than the state-of-the-art method. The measurement of the concentration of CA125 in the OC-plasma samples and the N-plasma samples by the clinically approved ELISA was not able to discriminate clearly OC-plasma samples from N-plasma samples, and two of ten N-plasma samples were higher than the upper reference limit.

### 4. Methods for measuring the concentration of markers

The concentrations of the markers in a biological sample such as blood, blood plasma, or blood serum obtained from a subject with suspected primary or relapsed OC are measured preferably by the aptamer-based SOMAscan® Proteomic Assay. Alternatively, the concentrations of these markers can be measured by using other methods like ELISA-based techniques, mass spectrometry, antibody- or aptamer-based microarrays or any other suitable quantification method. According to the method of measuring the concentrations the thresholds of each marker must been adjusted.

### Description of the Figures

**Figure 1** shows a graphical representation of the data for the significantly upregulated markers. The box pots depict medians (line within box), upper and lower quartiles (box), range (whiskers) and outliers (diamonds) constructed by using the Seaborn boxplot function.
**Figure 2** illustrates the separation of plasma samples from patients with OC (OC-plasma) and from subjects with non-malignant diseases (N-plasma) by a multi-marker score system consisting of adding up marker-wise normalized SOMAscan® Proteomic Assay units. The graph shows the distance (i.e. difference) between the minimum of all OC-plasma samples and the maximum of all N-plasma samples in relation to the number of combined markers. For example, the number 10 on the x-axis represents the combination of the first ten markers of Table 2. The dashed line shows the range of nearly linear increase.
**Figure 3** shows the distribution in OC-plasma and N-plasma samples for the top 10, 20, 30, 40, and 50 markers in Table 2. Units means the sum of marker-wise normalized SOMAscan® Proteomic Assay units as in Figure 1 and is the multi-marker score for patients with OC (OC-plasma) and subjects with non-malignant disease (N-plasma). Dots represent individual values; p-values were determined by two-tailed t-test.
**Figure 4** shows the distribution in OC-plasma and N-plasma samples for the top 5, 10, 20, 30, 40, 50, and 57 markers in Table 2. Score (multi-marker score) means the percentage of markers with concentrations above a predefined threshold as determined by the SOMAscan® Proteomic Assay. In this example, the threshold was defined as the 33^{th} percentile (lower tercile) of all 30 plasma samples (20 OC-plasma and 10 N-plasma).
**Figure 5** shows the distribution of marker levels measured for apolipoprotein A1 (APOA1), transferrin (TF), follicle-stimulating hormone-beta (FSHB), and choriogonadotropin-beta (CGB). Units were determined by SOMAscan® Proteomic Assay. Details see as in Figure 2.
**Figure 6** shows the distribution in OC-plasma and N-plasma samples for the refined combinations of 3 markers (A), 9 markers (B) and all 56 markers from Table 1 (except for TIMP1, excluded due to very low levels; C). Left panels in A-C: the scoring system used in Figure 4 was applied. Right panels in A-C: the scoring system used in Figure 3 was applied.
**Figure 7** shows that a clear discrimination of OC-plasma and N-plasma samples by OC125 measured by a clinically approved ELISA was not possible, and two of ten N-plasma samples were higher than the upper reference limit of 35 units/ml. These where thus very close to, or overlapped with OC plasma samples. The horizontal lines indicate the upper normal reference level (35 units/ml) and the pathological threshold for this clinically approved ELISA (65 units/ml).

## Claims

1. A method for diagnosing ovarian carcinoma (OC) in a biological sample from an individual with suspected OC or suspected relapsed OC, comprising the steps
(ix) measuring the concentration of at least the markers ASGR1, EPHA2, and SPON1 in the biological sample of said individual;
(x) comparing the measured concentrations from step (i) with predefined thresholds;
(xi) assigning a score of 1 for each marker which measured concentration is above its predefined threshold;
(xii) assigning a score of 0 for each marker which measured concentration is below its predefined threshold;
(xiii) adding the scores from step (iii) and (iv) to form a multi-marker score;
(xiv) dividing the multi-marker score from step (v) by the total number of measured markers from step (i);
(xv) multiplying the result of step (vi) by 100 to obtain a percentage;
(xvi) comparing this percentage to whether it is ≥60%;
where the said individual has OC, if the percentage is higher or equal than 60% and where the individual has no OC, if the percentage is lower than 60%.

2. A method for diagnosing ovarian carcinoma (OC) in a biological sample from an individual with suspected OC or suspected relapsed OC, according to claim 1, **characterized in that** in step (i) the concentrations of at least the markers ASGR1, EPHA2, SPON1, and THBS2 are determined in the biological sample of said individual.

3. A method for diagnosing ovarian carcinoma (OC) in a biological sample from an individual with suspected OC or suspected relapsed OC, according to claim 1, **characterized in that** in step (i) the concentrations of at least the markers ASGR1, EPHA2, SPON1, THBS2, and FUT5 are determined in the biological sample of said individual.

4. A method for diagnosing ovarian carcinoma (OC) in a biological sample from an individual with suspected OC or suspected relapsed OC, according to claim 1, **characterized in that** in step (i) the concentrations of at least the markers ASGR1, EPHA2, SPON1, THBS2, FUT5, EFNA4, CCDC80, FTH1, and C5a are determined in the biological sample of said individual.

5. A method for diagnosing ovarian carcinoma (OC) in a biological sample from an individual with suspected OC or suspected relapsed OC, according to claim 1, **characterized in that** in step (i) the concentrations of at least the markers ASGR1, SPON1, FUT5, EPHB2, SST, EPHA2, ANGPT2, C5a, EFNA4, SERPING1, PYY, CCL14, IL18BP, SPP1, SECTM1, TFPI, IGFBP2, FTH1, TNFRSF21, COLEC12, PRSS22, CCDC80, PDGFRA, HAMP, EFNA5, ENO2, STC1, SUMO2, COTL1, TNFSF15, HTRA2, VEGF, LBP, NRP1, C2, FABP3, RELT, FLRT2, C9, alpha-1-Antichymotrypsin, FSTL1, CST3, THBS2, TNFRSF8, SCARF1, B2M, Coagulation Factor IXab, GAS1, HSP90AB1, RBM39, NID1, ENO1, CRP, HSP90AA1, FSHB, and VWF are determined in the biological sample of said individual.

6. A method for diagnosing ovarian carcinoma (OC) in a biological sample from an individual with suspected OC or suspected relapsed OC, according to the previous claims 1 to 5, **characterized in that** the biological sample is blood plasma.

7. A method for diagnosing ovarian carcinoma (OC) in a biological sample from an individual with suspected OC or suspected relapsed OC according to the claims 1 to 6, **characterized in that** the determining of the concentration of said markers is performed by SOMAscan® Proteomic Assay.

8. A method for diagnosing OC in a biological sample from an individual with suspected OC or suspected relapsed OC according to the claims 1 to 6, **characterized in that** the determining of the concentration of said markers is performed by ELISA-based techniques, mass spectrometry, antibody- or aptamer-based microarrays, or any other suitable quantification method.

9. A device for diagnosing ovarian carcinoma (OC) in a biological sample from an individual according to claim 1, **characterized in that** the device comprises at least
- a measuring unit for measuring the concentration of at least the markers ASGR1, EPHA2, and SPON1 in the biological sample of said individual whereby this unit is capable to forward the measured concentrations to a data processing unit;
- a data processing unit which receives the measured concentrations from the measuring unit and which executes the steps (ii) to (viii) of claim 1 and forwards the results to the output unit;
- an input unit for data entry which is connectable to a database for the input of at least the predefined thresholds and which forwards data to the data processing unit;
- an output unit for data output which indicates OC, if the percentage is higher or equal than 60% and where the individual has no OC, if the percentage is lower than 60%.

10. Computer software to execute the method according to one of claim 1 to 8.
